# EUROPÄISCHE PATENTSCHRIFT

(11) **EP 0 570 946 B1**
(45) Veröffentlichungstag und Bekanntmachung des Hinweises auf die Patenterteilung: **27.12.1996**
(21) Anmeldenummer: 93108188.9
(22) Anmeldetag: 19.05.1993
(51) Int. Cl.: B65B 55/02, A61L 2/24

(54) **Sterilisiertunnel**
Sterilisation tunnel
Tunnel de stérilisation

(30) Priorität: 22.05.1992 DE 4217054
(43) Veröffentlichungstag der Anmeldung: 24.11.1993
(73) Patentinhaber: WILHELM BAUSCH, ROLF STRÖBEL, SIEGFRIED BULLINGER, Gesellschaft des Bürgerlichen Rechts, 74532 Ilshofen (DE)
(72) Erfinder: Dünsbier, Willi, D-7186 Blaufelden (DE); Schöllmann, Wolfgang, D-7180 Crailsheim (DE); Bullinger, Siegfried, D-7174 Ilshofen (DE)
(74) Vertreter: Liska, Horst, Dr.-Ing.

(56) Entgegenhaltungen:
- EP-A- 0 206 096
- DE-A- 3 321 195
- DE-A- 4 131 258
- FR-A- 2 358 163
- US-A- 5 022 165

## Beschreibung

Die Erfindung betrifft einen Sterilisiertunnel, umfassend einen Durchlaufofen, eine durch den Durchlaufofen hindurchführende Transporteinrichtung für zu sterilisierende Gegenstände, wenigstens je eine mittels zumindest eines Reinluftfilters einen laminaren Reinluft-Strömungsvorhang quer zur Durchlaufrichtung des Durchlaufofens erzeugende Filterschleuse am Eingang und am Ausgang des Durchlaufofens und eine insbesondere die Strömungsgeschwindigkeit einer durch den Durchlaufofen vom Ausgang zum Eingang verlaufenden Luftströmung regelnde oder minimierende Regelschaltung, deren Stellglied die Größe des Luftdurchsatzes und/oder des Luftdrucks im Bereich des Reinluft-Strömungsvorhangs der ausgangsseitigen Filterschleuse steuert.

An die Sterilisierung von Gegenständen des pharmazeutischen Bereichs, beispielsweise Ampullen oder dergleichen, werden außerordentlich hohe Sterilitätsanforderungen gestellt. So ist es aus der DE-A-37 34 830 bekannt, am Eingang und am Ausgang eines nach dem Strahlungswärmeprinzip arbeitenden Durchlaufofens Filterschleusen anzuordnen, die vor dem Eingang bzw. Ausgang des mittels eines Förderbands kontinuierlich horizontal beschickten Durchlaufofens vertikale laminare Reinluft-Strömungsvorhänge bilden. Die Filterschleusen umfassen Hochleistungs-Schwebstoffilter, die die Partikelzahlen in der Filterabluft auf sehr geringe Werte mindern. Der Luftdruck im Bereich der eingangsseitigen und ausgangsseitigen Filterschleusen sowie im Strahlungswärmebereich des Durchlaufofens wird so gesteuert, daß er von der eingangsseitigen Filterschleuse zur ausgangsseitigen Filterschleuse zunimmt und sich eine sehr geringe, vorzugsweise ebenfalls laminare Luftströmung vom Ausgang zum Eingang ergibt. Die der Förderrichtung entgegengesetzte Luftströmung sorgt für eine weitere Minderung der Partikelzahlen auf der Ausgangsseite des Sterilisiertunnels.

Bei dem aus der DE-A-37 34 830 bekannten Sterilisiertunnel ist zur Regelung der Strömungsgeschwindigkeit der vom Ausgang zum Eingang führenden Luftströmung ein Druckregelkreis vorgesehen, dessen Stellglied den Luftdurchsatz der ausgangsseitigen Filterschleuse abhängig von dem mittels eines Drucksensors im Durchlaufofen gemessenen Luftdrucks steuert. Da bereits geringe Druckschwankungen vergleichsweise hohe Schwankungen der Strömungsgeschwindigkeit verursachen, ist ein vergleichsweise empfindlicher Drucksensor erforderlich. Darüberhinaus beeinflussen Luftdruckschwankungen des üblicherweise an den Sterilisiertunnel ausgangsseitig anschließenden Reinluftraums die Regeleigenschaften, so daß es zu Regelschwankungen kommen kann. Zu Regelschwankungen kann es auch kommen, wenn sich der Beladungszustand des durch den Durchlaufofen hindurchführenden Förderbands ändert, da sich abhängig vom Beladungszustand der Öffnungsquerschnitt am Eingang und Ausgang des Durchlaufofens ändert. Schwankungen der Strömungsgeschwindigkeit im Durchlaufofen beeinflussen darüberhinaus die Betriebstemperatur des Durchlaufofens. Der bekannte Sterilisiertunnel umfaßt deshalb zusätzlich eine auf die Strahlungsheizelemente des Durchlaufofens wirkende Temperaturregelung.

Aus DE-41 31 258 A1 ist ein weiterer Sterilisiertunnel bekannt, bei welchem die zu sterilisierenden Gegenstände auf einem Transportband zuerst durch einen Heizteil des Sterilisiertunnels und dann durch einen Abkühlteil gefördert werden. In dem Heizteil wird die ggf. kontinuierlich durch Frischluft ergänzte, durch Infrarotstrahler erhitzte Luft in einem Umluftkreis geführt, während der Kühlteil nach dem Zuluft-Abluft-Prinzip arbeitet. Die Abluftleistung des Kühlteils wird durch einen verstellbaren Abluftventilator bestimmt, dem über ein motorisch stellbares Klappenventil Nebenluft zugeführt werden kann. Ein Teil der Luftströmung des Kühlteils kann über einen Sammelkanal in den Umluftkreislauf des Heizteils eingeführt werden. In dem Umluftkreislauf des Heizteils ist ein Drucksensor einer Druckregelschaltung angeordnet, die über eine zweite motorisch verstellbare, in einem Abluftweg angeordnete Klappe den Druck in dem Umluftkreislauf des Heizteils konstant hält. In dem Umluftkreislauf des Heizteils ist ferner ein Temperatursensor einer Temperaturregelschaltung angeordnet, die die erstgenannte, motorisch gesteuerte Stellklappe so einstellt, daß, die Temperatur der Umluftströmung in dem Heizteil konstant bleibt. Die Sensoren der beiden Regelschaltungen sind hierbei auf der Lufteintrittsseite eines in dem Umluftkreislauf angeordneten Partikelfilters angeordnet. Mit Hilfe der beiden Regelkreise wird erreicht, daß die aus dem Umluftkreislauf des Heizteils abgezweigte Abluft zur Energierückgewinnung über einen Wärmetauscher geführt werden kann.

Es ist Aufgabe der Erfindung, einen Sterilisiertunnel zu schaffen, der es erlaubt, die Betriebstemperatur im Durchlaufofen besser als bisher konstant zu halten und zugleich hohen Anforderungen an die Sterilität genügt.

Ausgehend von dem eingangs erläuterten Sterilisiertunnel wird diese Aufgabe erfindungsgemäß dadurch gelöst, daß die mittels ihres Stellglieds die Größe des Luftdurchsatzes und/oder Luftdrucks im Bereich des Reinluft-Strömungsvorhangs der ausgangsseitigen Filterschleuse steuernde Regelschaltung als Temperaturregelschaltung ausgebildet ist, die mittels eines Temperatursensors ein der Lufttemperatur im Bereich des Eingangs des Durchlaufofens entsprechendes Ist-Signal erzeugt und das Stellglied der ausgangsseitigen Filterschleuse abhängig von einem Vergleich des Ist-Signals mit einem Soll-Signal steuert. Mittels einer solchen ggf. zusätzlich zu einer Temperaturregelung der Heizeinrichtungen des Durchlaufofens vorgesehenen Temperaturregelschaltung kann die Strömungsgeschwindigkeit vom Ausgang zum Eingang des Durchlaufofens auf einem gewünschten ggf. sehr niedrigen Wert gehalten werden. Die Temperaturregelschaltung spricht sehr rasch an, neigt aber trotzdem nicht zu Regelschwingungen, selbst wenn es beispielsweise bei einem Beladungswechsel oder bei Lücken in der Beladung der durch den Durchlaufofen hindurchführenden Transporteinrichtung zu Strömungsgeschwindigkeitsschwankungen am Eingang oder Ausgang des Durchlaufofens kommen sollte. Da der Temperatursensor am Eingang des Durchlaufofens vorgesehen ist, werden Strömungsschwankungen, die den Austrag erhitzter Luft aus dem Durchlaufofen ändern, unmittelbar erfaßt.

Bei dem Soll-Signal kann es sich um einen fest vorgegebenen Signalwert handeln. Um Änderungen der Umgebungsparameter Rechnung tragen zu können, wird jedoch das Soll-Signal zweckmäßiauf ein Referenzsignal bezogen, das von einem außerhalb des Durchlaufofens angeordneten weiteren Temperatursensor erzeugt wird. Die Temperaturregelschaltung berücksichtigt das Referenzsignal zweckmäßigerweise dadurch, daß sie jeweils die Differenz zwischen Ist-Signal und Referenzsignanl mit dem vorgegebenen Soll-Signal vergleicht. Der weitere Temperatursensor kann beispielsweise die Lufttemperatur im Luftweg des Reinluftfilters der eingangsseitigen Filterschleuse und hier insbesondere im Bereich der Lufteintrittsseite des Reinluftfilters erfassen. Eine höhere Genauigkeit der Temperaturregelung wird jedoch erreicht, wenn der weitere Temperatursensor die Umgebungstemperatur des Sterilisiertunnels insbesondere im Bereich außerhalb des Durchlaufofens oder der eingangsseitigen Filterschleuse erfaßt.

Untersuchungen haben gezeigt, daß die Änderungsgeschwindigkeit, mit der die Temperatur im Strahlungswärmebereich des Durchlaufofens abnimmt in der Regel größer ist als die Änderungsgeschwindigkeit bei einer Temperaturerhöhung. Dies ist darauf zurückzuführen, daß bei einer Erhöhung der Luftströmungsgeschwindigkeit im Durchlaufofen die erhitzte Luft vergleichsweise rasch aus dem Strahlungswärmebereich ausgetragen wird, während bei Erniedrigung der Strömungsgeschwindigkeit der Temperaturanstieg nur entsprechend der Heizleistung des Durchlaufofens und damit vergleichsweise langsam zunehmen kann. Um das Regelverhalten der Temperaturregelschaltung verbessern zu können, ist in einer bevorzugten Ausgestaltung der Erfindung vorgesehen, daß dem das Ist-Signal erzeugenden Temperatursensor temperaturabhängig steuerbare Temperiermittel zugeorndet sind, die das Verhältnis der Abkühlrate, mit der der Temperatursensor einer Erniedrigung der Lufttemperatur folgt zur Erwärmungsrate, mit der der Temperatursensor einer Erhöhung der Lufttemperatur folgt, erhöhen. Ein derartiger Temperatursensor hat damit unterschiedliche Ansprechzeiten abhängig von der Änderungsrichtung der Temperatur. Bei sich erniedrigender Temperatur spricht der Temperatursensor rascher an, als bei sich erhöhender Temperatur. Regelschwingungen lassen sich auf diese Weise trotz des im Einzelfall vergleichsweise großen Abstands zwischen Temperaturmeßort am Eingang des Durchlaufofens und dem Stellort am Ausgang des Durchlaufofens sicher verhindern.

Unterschiedliche Ansprechzeitkonstanten bei Erwärmung und Abkühlung lassen sich beispielsweise dadurch erreichen, daß bei sich erhöhender Lufttemperatur der Temperatursensor zusätzlich durch Heizmittel erwärmt oder bei sich erniedrigender Temperatur zusätzlich durch Kühlmittel gekühlt wird. Bei den Heizmitteln kann es sich beispielsweise um eine elektrische und damit in einfacher Weise steuerbare Heizeinrichtung an dem Temperatursensor handeln. Um die Wärmekapazität des Temperatursensors jedoch nicht durch zusätzlich an dem Temperatursensor anzuordnende Körper zu erhöhen, ist in einer bevorzugten Ausgestaltung vorgesehen, daß der Temperatursensor in einem Meßluft führenden Meßkanal angeordnet ist, der im Strömungsweg vor dem Temperatursensor mit einem Luft mit von der Meßlufttemperatur abweichender Temperatur führenden Nebenkanal der Temperiermittel verbunden ist, wobei der Querschnitt des Nebenkanals mittels eines Stellantriebs, beispielsweise eines elektromagnetischen Sperrventils änderbar ist. In einer solchen Ausgestaltung kann die Temperatur der am Temperatursensor vorbeigeführten Meßluft sehr rasch und trägheitsarm in definierter Weise variiert werden.

Besonders gleichmäßig reproduzierbare Ansprecheigenschaften des Temperatursensors werden erreicht, wenn der Nebenkanal als insbesondere mit der Umgebungsluft des Sterilisiertunnels verbundener Kühlluft-Zuführungskanal ausgebildet ist, und der Stellantrieb temperaturabhägig so steuerbar ist, daß er den Kühlluftdurchsatz bei sich verringernder Meßlufttemperatur erhöht, verglichen mit dem Kühlluftdurchsatz bei sich erhöhender Meßlufttemperatur. Zur Adaptierung der Temperaturregelschaltung an die Betriebsweise und die Parameter des Durchlaufofens mündet in den Meßkanal im Strömungsweg vor dem Temperatursensor und insbesondere vor der Verbindungsstelle mit dem Nebenkanal zweckmäßigerweise ein weiterer Kühlluftzuführungskanal, der gleichfalls bevorzugt mit der Umgebungsluft des Sterilisiertunnels verbunden ist. Der Meßkanal oder/und zumindest einer der Kühlluftzuführungskanäle kann hierzu ein Durchsatzjustierventil enthalten. Durch Justierung des Durchsatzes des Meßkanals einerseits und zumindest eines der Kühlluftzuführungskanäle andererseits läßt sich sowohl das Verhältnis als auch die absolute Größe der Ansprechgeschwindigkeiten für die beiden Temperaturänderungsrichtungen justiern.

Die Erfindung eignet sich insbesondere für einen Sterilisiertunnel, dessen Durchlaufofen in einem Umluftkreislauf angeordnete Heizeinrichtungen hat, wobei der Umluftkreislauf quer zur Transportrichtung durch den Durchlaufofen geführt ist. Sie eignet sich aber in der gleichen Weise auch für Strahlungshitze-Durchlauföfen.

Im folgenden soll die Erfindung anhand einer Zeichnung näher erläutert werden. Hierbei zeigt:
- Figur 1: eine schematische Darstellung eines Ausführungsbeispiels eines erfindungsgemäßen sterilisiertunnels;
- Figuren 2a und 2b: Zeitdiagramme zur Erläuterung der Funktionsweise einer Temperatursensoranordnung des Sterilisiertunnels und
- Figur 3: eine schematische Darstellung der Temperatursensoranordnung.

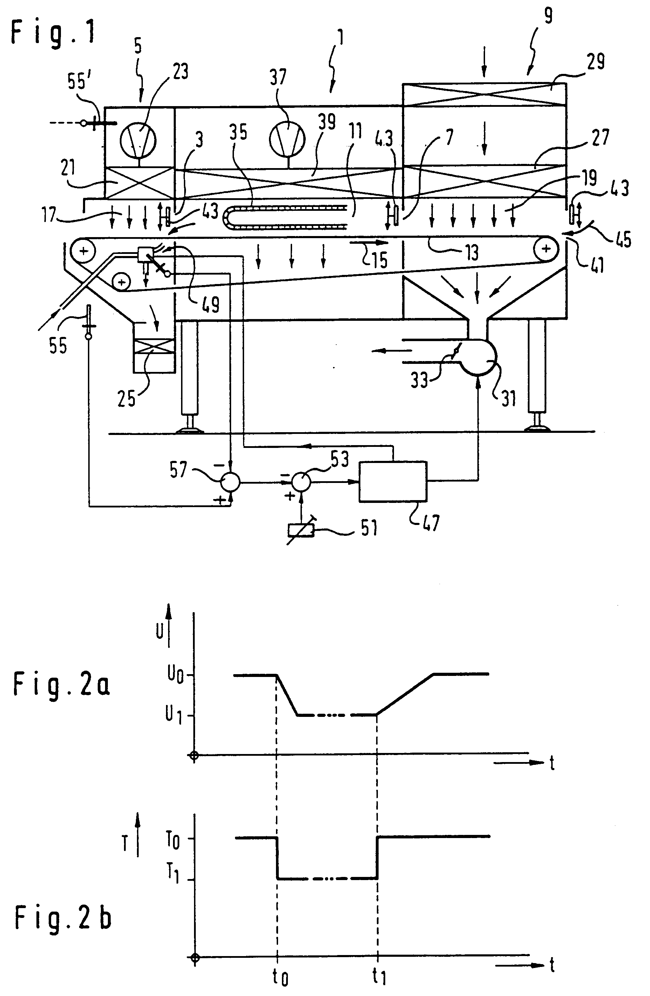

Der in Figur 1 schematisch dargestellte Sterilisiertunnel umfaßt einen Durchlaufofen 1, vor dessen Eingangsöffnung 3 wenigstens eine eingangsseitige Filterschleuse 5 angeordnet ist und an dessen Ausgangsöffnung 7 zumindest eine ausgangsseitige Filterschleuse 9 anschließt. Durch die Filterschleusen 5, 9 und die bei 11 dargestellte Sterilisierkammer des Durchlaufofens 1 erstreckt sich ein Endlos-Transportband 13, das die zu sterilisierenden Gegenstände, beispielsweise pharmazeutische Artikel, wie z. B. Ampullen, Spritzenkörper oder dergleichen in horizontaler, durch einen Pfeil 15 angedeuteter Transportrichtung der Reihe nach durch die Filterschleuse 5, den Durchlaufofen 1 und die Filterschleuse 9 bewegt. Das in Figur 1 einteilig dargestellte Transportband 13 kann in Transportrichtung aus mehreren Segmenten bestehen, die die Gegenstände lediglich über Abschnitte des Transportwegs transportieren. Die Transportfläche des Transportbands 13 hat Loch- oder Gitterstruktur und ermöglicht im wesentlichen im gesamten Transportweg einen vertikalen Luftdurchtritt an den zu sterilisierenden Gegenständen vorbei.

Die Filterschleusen 5, 9 arbeiten nach dem "laminar-flow-Prinzip", d.h. sie erzeugen vor der Eingangsöffnung 3 bzw. hinter der Ausgangsöffnung 7 laminar strömende Reinluftvorhänge 17, 19 mit vertikaler Strömungsrichtung, hier von oben nach unten. Die Filterschleusen 5, 9 umfassen Hochleistungs-Schwebstoffilter, über die die Luft der Reinluft-Strömungsvorhänge 17, 19 geführt wird. Die eingangsseitige Filterschleuse 5 umfaßt ein dem Strömungsvorhang 17 vorgeschaltetes Hauptfilter 21. Ein Gebläse 23 in einem nicht näher dargestellten Umluftkanal zirkuliert die Reinluft über das Hauptfilter 21, den Strömungsvorhang 17 und ggf. ein oder mehrere dem Strömungsvorhang 17 nachfolgende Vorfilter 25.

Die Filterschleuse 9 arbeitet im dargestellten Ausführungsbeispiel nach dem Zuluft-Abluft-Prinzip, kann jedoch entsprechend der Filterschleuse 5 auch nach dem Umluft-Prinzip arbeiten. Die Filterschleuse 5 umfaßt wiederum mehrere Hochleistungs-Schwebstoffilter, hier mit einem dem Strömungsvorhang 19 unmittelbar vorgeschalteten Hauptfilter 27, dem auf der Zuluftseite ein Vorfilter 29 vorgeschaltet ist. Auf der Abluftseite des Strömungsvorhangs 19 ist ein Sauggebläse 31 angeordnet, dessen Förderleistung durch Steuerung seines Elektromotors oder aber einer bei 33 angedeuteten Drosselklappe steuerbar ist.

Der Durchlaufofen 1 umfaßt ein elektisches Heizregister 35, über das ein Gebläse 37 die auf Sterilisiertemperatur von beispielsweise 320° C erhitzte Luft in einem Umluft-Kreislauf zirkulieren läßt. Der Umluft-Kreislauf führt von dem Gebläse 37 über ein heißluftbeständiges Hochleistungs-Schwebstoffilter 39 entsprechend der Strömungsrichtung der Strömungsvorhänge 17, 19 von oben nach unten durch den Sterilisierkanal 11, in welchem auch die Heizregister 35 angeordnet sind. Um eine Auskühlung des Durchlaufofens 21 weitgehend zu verhindern sind an der Eingangsöffnung 3 und der Ausgangsöffnung 7 des Durchlaufofens 1 wie auch an einer Ausgangsöffnung 41 der ausgangsseitigen Filterschleuse 9 vertikal bewegliche Schieber 43 angeordnet, die ggf. automatisch gesteuert die Durchtrittsöffnung auf die Höhe der auf dem Transportband stehenden, zu sterilisierenden Gegenstände begrenzen. Eine bevorzugte Steuerung bewirkt, daß die Schieber 43 bei leerem Tunnel ganz geschlossen sind und kurz vor Eintreffen der zu sterilisierenden Gegenstände, zum Beispiel Flaschen, auf ein vorbestimmtes Maß geöffnet werden sowie bei Arbeitsende, das heißt beim Leerfahren unmittelbar nachdem die letzten Gegenstände den Schieber durchlaufen haben, dieser geschlossen wird. Die Schieber können auch bei einer Unterbrechung des Flaschenstroms geschlossen bzw. danach geöffnet werden.

Im Betrieb schließt sich an die Ausgangsöffnung 41 des Sterilisiertunnels ein normalerweise unter einem geringen Überdruck stehender Sterilraum an, in welchem die sterilisierten Gegenstände weiterverarbeitet werden, beispielsweise mit sterilen Produkten befüllt werden. Der Überdruck in dem Sterilraum sorgt dafür, daß unter anderem über den sterilisiertunnel Reinluft aus dem Sterilraum ausschließlich abströmen kann, um ungewollte Kontaminierung zu vermeiden. Diese in Figur 1 durch einen Pfeil 45 angedeutete ausgangsseitige Zuluft wird durch das mittels einer Regelschaltung 47 in seiner Saugleistung gesteuerte Sauggebläse 31 abgesaugt. Die Regelschaltung 47 sorgt durch Einstellen der Strömungsgeschwindigkeit und/oder des Luftdrucks im Strömungsvorhang 19 dafür, daß Luftströmungen sowohl an der Eingangsöffnung 3 als auch an der Ausgangsöffnung 7 des Sterilisierkanals 11 weitgehend verringert werden oder aber zumindest auf einem konstanten Wert gehalten werden, um zu verhindern, daß die auf der Sterilisiertemperatur gehaltene Heißluft aus dem Durchlaufofen 11 ausgetragen wird. Durch Austragen von Heißluft aus dem Durchlaufofen 1 werden nicht nur die Betriebskosten des Sterilisiertunnels erhöht, sondern es kann auch zu unerwünschten Temperaturschwankungen im Sterilisierkanal 11 kommen. Die ausgetragene Heißluft heizt darüber hinaus in unerwünschter Weise die Umgebung auf.
Die das Sauggebläse 31 steuernde Regelschaltung 47 ist Bestandteil eines Temperatur-Regelkreises, der die Temperatur im Eingangsbereich des Sterilisierkanals 11 mittels einer nachfolgend noch näher erläuterten Temperatursensoranordnung 49 erfaßt und durch Bildung der Differenz zwischen einem aus einer Stufe 51 zugeführten Soll-Wert-Signal und dem durch Messung der Temperatur im Eingangsbereich des Sterilisierkanals 11 gewonnenen Ist-Signals ein Fehler- bzw. Regelabweichungssignal für die Regelschaltung 47 mittels eines Vergleichers 53 oder dergleichen erzeugt. Um den Einfluß der Umgebungsparameter des Sterilisierkanals 11 ausgleichen zu können, wird als Regelgröße nicht unmittelbar das die Temperatur im Eingangsbereich des Sterilisierkanals 11 repräsentierende Ausgangssignal der Temperatursensoranordnung 49 mit dem Sollwertsignal verglichen, sondern die Differenz der Temperatur-Ausgangssignale eines weiteren Temperatursensors 55 und der Temperatursensoranordnung 49. Eine die Differenz bildende Stufe ist bei 57 angedeutet. Der Temperatursensor 55 erfaßt zweckmäßigerweise die Außenlufttemperatur in der Umgebung der eingangsseitigen Filterschleuse 5, kann aber auch, wie bei 55' angedeutet die Temperatur der im Umluftkreislauf der eingangsseitigen Filterschleuse 5 zirkulierenden Reinluft insbesondere vor deren Eintritt in das Hauptfilter 21 messen.

Die von der Temperatursensoranordnung 49 gemessene Temperatur ist ein Maß für die Temperatur der im Durchlaufofen 1 zirkulierenden Heißluft, die im Eingangsbereich indirekt auch ein Maß für die Strömungsgeschwindigkeit der durch die Eingangsöffnung 3 zu- oder abfließenden Luftströmung ist. Entsprechend dieser indirekt gemessenen eingangsseitigen Luftströmung regelt die Regelschaltung 47 die Förderleistung des Sauggebläses 31 und damit indirekt den Luftdruck im Bereich des Strömungsvorhangs 19 der ausgangsseitigen Filterschleuse 9. Im Endeffekt regelt die Regelschaltung 47 damit die Druckdifferenz zwischen Eingangsseite und Ausgangsseite des Durchlaufofens 1 und damit die Größe der durch den Sterilisierkanal 11 strömenden Luftströmung. Die vom Sensor 49 gemessene Luft wird hierbei nicht direkt aus der Zone 11 entnommen, sondern aus der Zone 5 in der Nähe des Übergangs zu Zone 11. Gemessen wird also ein Luftgemisch aus Zone 5 und 11. Je höher die Querströmung ist, desto höher ist auch die durch den Sensor 49 gemessene Temperatur.

Temperatursensoren, die die Temperatur durch unmittelbaren Wärmekontakt mit der Luft messen, haben eine Ansprechzeitkonstante, die beispielsweise durch die Wärmekapazität des Sensorkörpers bestimmt wird. Um Temperaturänderungen der Luft möglichst rasch erfassen zu können, soll die Ansprechzeitkonstante möglichst klein sein. Andererseits neigen Temperaturregelkreise mit rasch ansprechendem Temperatursensor zu Regelschwingungen. Bei dem Sterilisiertunnel mindert von außen einströmende kalte Luft die Temperatur im Sterilisierkanal 11 sehr rasch. Andererseits benötigt das Heizregister 35 vergleichsweise viel Zeit, um die Temperatur nachfolgend wieder anzuheben. Weiterhin ist der Abstand zwischen Temperatursensoranordnung 49 und dem als Stellglied des Temperaturregelkreises dienenden Sauggebläse 31 vergleichsweise groß, so daß sich totzeitbedingt Regelschwingungen der nicht näher dargestellten, jedoch vorhandenen, die Heizregister 35 steuernden Temperaturregeleinrichtungen des Durchlaufofens 1 ergeben können.

Um Regelschwingungen der vorstehend erläuterten Art zu vermeiden ist die Temperatursensoranordnung 49 so ausgebildet, daß sie auf eine Temperaturerniedrigung rascher anspricht als auf eine Temperaturerhöhung. Wie die Figuren 2a und 2b zeigen nimmt die Ausgangsspannung U der Temperatursensoranordnung bei einer sprunghaften Abnahme der gemessenen Temperatur T vom Wert T₀ auf den Wert T₁ (Zeitpunkt t₀) vergleichsweise rasch vom Wert U₀ auf dem Wert U₁ ab. Nimmt jedoch die Temperatur T wie für den Zeitpunkt t₁ dargestellt, vom Wert T₁ sprunghaft auf den Wert T₀ zu, so steigt die Ausgangsspannung vergleichsweise langsam vom Wert U₁ auf den Wert U₀ an.

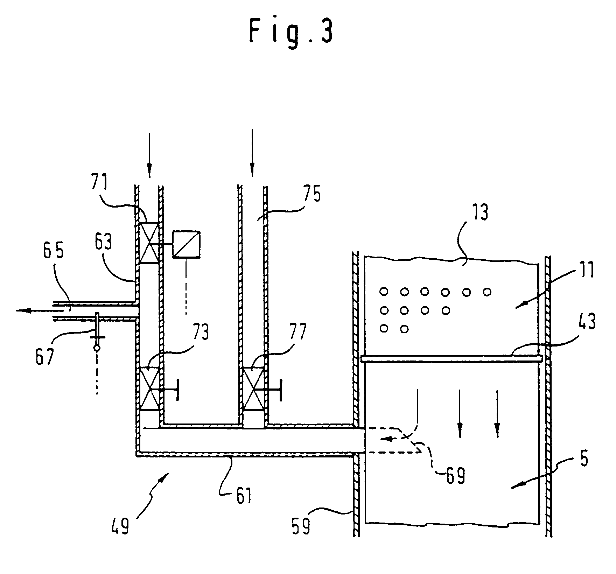

Figur 3 zeigt schematisch Einzelheiten der Temperatursensoranordnung 49. Die Temperatursensoranordnung 49 umfaßt ein zweckmäßigerweise durch eine Seitenwand 59 des in Figur 3 in Draufsicht auf das Transportband 13 dargestellten Einlaufzone 5 des Sterilisierkanals 11 hindurchreichendes Meßrohr 61, das sich außerhalb der Einlaufzone 5 in einen Kühlluftkanal 63 und einen weiteren Meßrohrabschnitt 65 verzweigt. Der Meßrohrabschnitt 65 hat einen gegenüber dem Kühlluftkanal 63 und dem Meßrohr 61 verengten Querschnitt und ist mit seinem Ende mit einer Stelle innerhalb der Filterschleuse 5 verbunden, an der, verglichen mit dem Luftdruck im Eingangsbereich des Sterilisierkanals 11 ein niedrigerer Luftdruck herrscht, beispielsweise mit der Ausgangsseite der Luftschleuse 17 oder der Eingangsseite des Gebläses 23. Ein Temperatursensor 67, der das Temperatur-Ausgangssignal der Sensoranordnung 49 liefert, mißt die Temperatur der aufgrund des Druckunterschieds durch den Meßrohrabschnitt 65 gesaugten Luft.

Zur Erleichterung des Lufteintritts in das Meßrohr 61 kann dessen in die Einlaufzone 5 ragendes Ende 69 auf der gegen die Luftströmungsrichtung weisenden Seite abgeschrägt oder gegen die Luftströmungsrichtung abgebogen sein. Zweckmäßigerweise befindet sich das Ende 69 zwischen dem vorlaufenden und dem rücklaufenden Teil des Transportbands 13.

Der Kühlluftkanal 63 ist mit einer Stelle der Umgebung des Durchlaufofens 1 verbunden, die sich auf einem niedrigeren Temperaturniveau als der Sterilisierkanal 11 befindet. Insbesondere mündet der Kühlluftkanal 63 an einer Stelle, deren Temperatur von dem Temperatursensor 55 erfaßt wird, im Ausführungsbeispiel der Figur 1 also insbesondere der Atmosphäre außerhalb der eingansseitigen Filterschleuse bzw. Einlaufzone 5. In dem Kühlluftkanal 63 ist ein Magnetventil 71 angeordnet, das abhängig von der Änderungsrichtung der vom Temperatursensor 67 erfaßten Luft steuerbar ist. Im Ausführungsbeispiel der Figur 1 liefert die Regelschaltung 47 ein Steuersignal, das bei abnehmender Lufttemperatur das Magnetventil 71 öffnet und bei zunehmender Lufttemperatur sperrt. Bei abnehmender Lufttemperatur wird über den Kühlluftkanal 63 zusätzlich zu der über das Meßrohr 61 angesaugten heißen Meßluft kühle Luft aus der Außenumgebung angesaugt, wodurch der Temperatursensor 67 zusätzlich gekühlt wird und damit rascher der abnehmenden Temperatur der Meßluft folgen kann. Die Anordnung kann aber auch so getroffen sein, daß bei rascher Zunahme der Lufttemperatur am Sensor 67 die Regelschaltung 47 ein das Magnetventil 71 öffnendes Steuersignal liefert, so daß durch die Kühlluftströmung einer übermäßigen Temperaturzunahme des Sensors 67 entgegen gewirkt wird. Bei rasch abnehmender Lufttemperatur am Sensor 67 bleibt das Magnetventil 71 geschlossen. Gegebenenfalls kann als Temperiermittel eine Zusatzheizung zugeschaltet werden. Mittels eines im Meßrohr 69 angeordneten, stufenlos einstellbaren Justierventils 73 kann das Mischungsverhältnis von Meßluft zu Kühlluft justiert werden. Auf der dem Meßrohrabschnitt 65 abgewandten Seite mündet in das Meßrohr 61 ein weiterer Kühlluftkanal 75, der ebenfalls ein stufenlos einstellbares Justierventil 77 enthält. Der Kühlluftkanal 75 der an der selben Stelle wie auch der Kühlluftkanal 63 münden kann, mischt der heißen aus der Einlaufzone 5 angesaugten Meßluft kühle Luft zu. Durch Einstellen der Justierventile 63, 77, bei welchen es sich beispielsweise um Kugelventile oder dergleichen handeln kann, kann sowohl der Absolutwert als auch das Verhältnis der Absolutwerte der Ansprechzeitkonstanten für zunehmende bzw. abnehmende Lufttemperatur variiert werden.

Bei Arbeitsanfang, das heißt bei kaltem Tunnel, ist die Regelschaltung 47 nicht aktiv und der Abluftventilator 31 wird auf eine fest vorgegebene Grunddrehzahl beschleunigt. Bei Erreichen der vorbestimmten Sterilisertemperatur von zum Beispiel 320 °C oder bei Erreichen einer vorbestimmten Differenztemperatur an den Sensoren 67 und 55 wird die Regelschaltung 47 aktiv auf Drehzahlregelung umgeschaltet.

Bei Arbeitsende bleibt die Regelschaltung 47 so lange aktiv, bis die vorgegebene Differenztemperatur an den Sensoren 67 und 55 nicht mehr gehalten werden kann, das heißt die Heizleistung stark abgefallen ist, dann erfolgt eine Umschaltung auf die fest vorgegebene Grunddrehzahl des Abluftventilators 31.

## Patentansprüche

1. Sterilisiertunnel, umfassend
einen Durchlaufofen (1), eine durch den Durchlaufofen (1) hindurchführende Transporteinrichtung (13) für zu sterilisierende Gegenstände, wenigstens je eine mittels zumindest eines Reinluftfilters (21, 27) einen laminaren Reinluft-Strömungsvorhang (17, 19) quer zur Durchlaufrichtung des Durchlaufofens (1) erzeugende Filterschleuse (5, 9) am Eingang (3) und am Ausgang (7) des Durchlaufofens (1) und einer insbesondere die Strömungsgeschwindigkeit einer durch den Durchlaufofen (1), vom Ausgang (7) zum Eingang (3) verlaufenden Luftströmung regelnde oder minimierende Regelschaltung (47), deren Stellglied die Größe des Luftdurchsatzes und/oder des Luftdrucks im Bereich des Reinluft-Strömungsvorhangs (19) der ausgangsseitigen Filterschleuse (9) steuert,
**dadurch gekennzeichnet**,
daß die Regelschaltung (47) als Temperaturregelschaltung ausgebildet ist, die mittels eines Temperatursensors (67) ein der Lufttemperatur im Bereich des Eingangs (3) des Durchlaufofens (1) entsprechendes Ist-Signal erzeugt und das Stellglied (31) der ausgangsseitigen Filterschleuse (9) abhängig von einem Vergleich des Ist-Signals mit einem Soll-Signal steuert.

2. Sterilisierungstunnel nach Anspruch 1,
**dadurch gekennzeichnet**,
daß zur Erzeugung eines Referenzsignals ein die Lufttemperatur außerhalb des Durchlaufofens (1) erfassender, weiterer Temperatursensor (55) vorgesehen ist und die Temperaturregelschaltung (47) das Stellglied (31) abhängig von dem Vergleich der Differenz zwischen dem Ist-Signal und dem Referenzsignal mit dem Soll-Signal steuert.

3. Sterilisiertunnel nach Anspruch 2,
**dadurch gekennzeichnet**,
daß der weitere Temperatursensor (55) die Lufttemperatur im Luftweg des Reinluftfilters (21) der eingangsseitigen Filterschleuse, insbesondere im Bereich der Lufteintrittseite des Reinluftfilters (21) erfaßt.

4. Sterilisiertunnel nach Anspruch 2,
**dadurch gekennzeichnet**,
daß der weitere Temperatursensor (55) die Umgebungstemperatur des Sterilisiertunnels insbesondere im Bereich außerhalb des Durchlaufofens (1) oder der eingangsseitigen Filterschleuse (5) erfaßt.

5. Sterilisiertunnel nach einem der Ansprüche 1 bis 4,
**dadurch gekennzeichnet**,
daß dem die Lufttemperatur im Bereich des Eingangs (3) des Durchlaufofens (1) erfassenden Temperatursensor (67) temperaturabhängig steuerbare Temperiermittel (63, 71) zugeordnet sind, die das Verhältnis der Abkühlrate, mit der der Temperatursensor (67) einer Erniedrigung der Lufttemperatur folgt zur Erwärmungsrate, mit der der Temperatursensor (67) einer Erhöhung der Lufttemperatur folgt, erhöhen.

6. Sterilisierungstunnel nach Anspruch 5,
**dadurch gekennzeichnet**,
daß der Temperatursensor (67) in einem Meßluft führenden Meßkanal (61, 65) angeordnet ist, der im Strömungsweg vor dem Temperatursensor (67) mit einem Luft mit von der Meßlufttemperatur abweichender Temperatur führenden Nebenkanal (63) der Temperiermittel (63, 71) verbunden ist, dessen Querschnitt mittels eines Stellantriebs (71) änderbar ist.

7. Sterilisierungstunnel nach Anspruch 6,
**dadurch gekennzeichnet**,
daß der Nebenkanal als insbesondere mit der Umgebungsluft des Sterilisiertunnels verbundener Kühlluft-Zuführungskanal (63) ausgebildet ist und der Stellantrieb (71) temperaturabhängig so steuerbar ist, daß er den Kühlluftdurchsatz bei sich verringender Meßlufttemperatur erhöht, verglichen mit dem Kühlluftdurchsatz bei sich erhöhender Meßlufttemperatur.

8. Sterilisiertunnel nach Anspruch 7,
**dadurch gekennzeichnet**,
daß in den Meßkanal (61, 65) im Strömungsweg vor dem Temperatursensor (67) und insbesondere vor der Verbindungsstelle mit dem Nebenkanal (63) ein weiterer Kühlluftzuführungskanal (75) mündet.

9. Sterilisiertunnel nach Anspruch 7 oder 8,
**dadurch gekennzeichnet**,
daß der Meßkanal (61, 65) oder/und zumindest einer der Kühlluftzuführungskanäle (63, 75) ein Durchsatzjustierventil (73,77) enthält.

10. Sterilisiertunnel nach einem der Ansprüche 6 bis 9,
**dadurch gekennzeichnet**,
daß der Stellantrieb als die Luftströmung im Nebenkanal (63) wechselweise freigebendes oder sperrendes Magnet-Sperrventil (71) ausgebildet ist.

11. Sterilisiertunnel nach einem der Ansprüche 6 bis 10,
**dadurch gekennzeichnet**
daß der Meßkanal (61,65) strömungsabwärts des Temperatursensors (67) mit einer Stelle verbunden ist an der, verglichen mit dem Luftdruck im Bereich des Eingangs des Durchlaufofens (1) ein niedrigerer Luftdruck herrscht und insbesondere mit einer Stelle im Luftströmungsweg der eingangsseitgen Filterschleuse (5) verbunden ist.

## Claims

1. Sterilisation tunnel, comprising a continuous furnace (1), a transport device (13) for the objects to be sterilised which passes through the continuous furnace (1), at least one each of a filter channel (5, 9) producing by means of at least one pure air filter (21, 27) a laminar pure air flow curtain (17, 19) at right angles to the feed-through direction of the continuous furnace (1) at the input (3) and at the output (7) of the continuous furnace (1), and a control circuit (47) controlling or minimising in particular the flow rate of air flowing through the continuous furnace (1) from the output (7) to the input (3), the actuating element of which controls the throughflow of air and/or air pressure in the region of the pure air flow curtain (19) of the output side filter channel (9),
**characterised in that** the control circuit (47) is designed as a temperature control circuit which by means of a temperature sensor (67) produces an actual-signal corresponding to the air temperature in the region of the input (3) of the continuous furnace (1) and controls the actuating element (31) of the output side filter channel (9) depending on the result of a comparison of the actual signal with the nominal signal.

2. Sterilisation tunnel according to claim 1, **characterised in that** to produce a reference signal an additional temperature sensor (55) for detecting the air temperature outside the continuous furnace (1) is provided, and the temperature control circuit (47) controls the actuating element (31) depending on the result of a comparison of the difference between the actual signal and the reference signal with the nominal signal.

3. Sterilisation tunnel according to claim 2, **characterised in that** the additional temperature sensor (55) detects the air temperature in the airway of the pure air filter (21) of the input side filter channel, in particular in the region of the air inlet side of the pure air filter (21).

4. Sterilisation tunnel according to claim 2, **characterised in that** the additional temperature sensor (55) detects the surrounding temperature of the sterilisation tunnel, in particular in the region outside the continuous furnace (1) or the input-side filter channel (5).

5. Sterilisation tunnel according to one of claims 1 to 4, **characterised in that** temperature-controllable tempering means (63, 71) are assigned to the temperature sensor (67) detecting the air temperature in the region of the input (3) of the continuous furnace (1), which increase the ratio of the cooling rate, at which the temperature sensor (67) lowers air temperature, to the heating rate, at which the temperature sensor (67) increases air temperature.

6. Sterilisation tunnel according to claim 5, **characterised in that** the temperature sensor (67) is arranged in a flow measuring channel (61, 65) carrying measured air, which is connected in the flow path in front of the temperature sensor (67) with a secondary channel (63) of the tempering means (63, 71) carrying air that has a temperature different from the measured air temperature, the cross section of which can be adjusted by means of an actuating drive (71).

7. Sterilisation tunnel according to claim 6, **characterised in that** the secondary channel is designed as a cooling air supply channel (63) connected in particular to the surrounding air of the sterilisation tunnel, and the actuating drive (71) is controllable by temperature so that it increases the throughflow of cooling air with decreasing measured air temperature, relative to the throughflow of cooling air with increasing measured air temperature.

8. Sterilisation tunnel according to claim 7, **characterised in that** an additional cooling air supply channel (75) enters into the measuring channel (61, 65) in the flow path in front of the temperature sensor (67) and in particular in front of the connecting point with the secondary channel (63).

9. Sterilisation tunnel according to claim 7 or 8, **characterised in that** the measuring channel (61, 65) and/or at least one of the cooling air supply channels (63, 75) comprises a flow-rate adjusting valve (73, 77).

10. Sterilisation tunnel according to one of claims 6 to 9, **characterised in that** the actuating drive is designed as a solenoid stop valve (71) alternately opening or closing the airflow in the secondary channel (63).

11. Sterilisation tunnel according to one of claims 6 to 10, **characterised in that** the measuring channel (61, 65) is connected upstream of the temperature sensor (67) at a point at which there is lower air pressure compared to the air pressure in the region of the input of the continuous furnace (1) and is connected in particular with a point in the airflow path of the input-side filter channel (5).

## Revendications

1. Tunnel de stérilisation, comprenant un four continu (1), un dispositif de transport (13) passant à travers le four continu (1) pour des objets à stériliser, au moins un sas filtrant (5, 9), produisant au moyen d'au moins un filtre à air pur (21, 27) un rideau d'écoulement laminaire d'air pur (17, 19) transversalement à la direction de traversée du four continu (1), à l'entrée (3) et à la sortie (7) du four continu (1), et un circuit de régulation (47) régulant ou minimisant en particulier la vitesse d'écoulement d'un courant d'air traversant le four continu (1) de la sortie (7) à l'entrée (3), circuit dont l'élément de régulation commande la valeur du débit d'air et/ou de la pression de l'air au niveau du rideau d'écoulement d'air pur (19) du côté de sortie du sas filtrant (9), caractérisé en ce que le circuit de régulation (47) est conformé en circuit de régulation de température qui, au moyen d'un capteur de température (67), produit un signal de mesure correspondant à la température de l'air au niveau de l'entrée (3) du four continu (1) et commande l'élément de régulation (31) du côté de sortie du sas filtrant (9) en fonction d'une comparaison du signal de mesure avec un signal de consigne.

2. Tunnel de stérilisation selon la revendication 1, caractérisé en ce que, pour produire un signal de référence, il est prévu un autre capteur de température (55) déterminant la température de l'air à l'extérieur du four continu (1), et le circuit de régulation de température (47) commande l'élément de régulation (31) en fonction de la comparaison de la différence entre le signal de mesure et le signal de référence avec le signal de consigne.

3. Tunnel de stérilisation selon la revendication 2, caractérisé en ce que l'autre capteur de température (55) détermine la température de l'air sur la voie d'air du filtre à air pur (21) du côté d'entrée du sas filtrant, en particulier au niveau du côté d'entrée d'air du filtre à air pur (21).

4. Tunnel de stérilisation selon la revendication 3, caractérisé en ce que l'autre capteur de température (55) détermine la température ambiante du tunnel de stérilisation, en particulier au niveau de l'extérieur du four continu (1) ou du côté d'entrée du sas filtrant (5).

5. Tunnel de stérilisation selon l'une des revendications 1 à 4, caractérisé en ce qu'au capteur de température (67) qui détermine la température de l'air au niveau de l'entrée (3) du four continu (1) sont associés des moyens d'équilibrage de température (63, 71) pouvant être commandés en fonction de la température, qui augmentent le rapport de la vitesse de refroidissement à laquelle le capteur de température (67) suit un abaissement de la température de l'air à la vitesse de réchauffement à laquelle le capteur de température (67) suit une augmentation de la température de l'air.

6. Tunnel de stérilisation selon la revendication 5, caractérisé en ce que le capteur de température (67) est placé dans un canal de mesure (61, 65) traversé par de l'air de mesure, canal qui est relié, en amont du capteur de température (67), à un canal annexe (63) des moyens d'équilibrage (63, 71) traversé par de l'air à une température différente de la température de l'air de mesure, et dont la section peut être modifiée au moyen d'un mécanisme de réglage (71).

7. Tunnel de stérilisation selon la revendication 6, caractérisé en ce que le canal annexe est conformé en canal d'alimentation d'air de refroidissement (63) relié en particulier à l'air environnant le tunnel de stérilisation, et le mécanisme de réglage (71) peut être commandé en fonction de la température de manière telle qu'il augmente le débit en air de refroidissement lorsque la température de l'air de mesure diminue, par rapport au débit d'air de refroidissement lorsque la température de l'air de mesure augmente.

8. Tunnel de stérilisation selon la revendication 7, caractérisé en ce qu'un autre canal d'alimentation en air de refroidissement (75) débouche dans le canal de mesure (61, 65) sur la voie d'écoulement avant le capteur de température (67), et en particulier avant le point de raccordement avec le canal annexe (63).

9. Tunnel de stérilisation selon la revendication 7 ou 8, caractérisé en ce que le canal de mesure (61, 65) et/ou au moins l'un des canaux d'air de refroidissement (63, 75) contient une vanne de réglage de débit (73, 77).

10. Tunnel de stérilisation selon l'une des revendications 6 à 9, caractérisé en ce que le mécanisme de réglage est conformé en vanne de blocage magnétique (71) bloquant ou libérant alternativement l'écoulement d'air dans le canal annexe (63).

11. Tunnel de stérilisation selon l'une des revendications 6 à 10, caractérisé en ce que le canal de mesure (61, 65) est relié en aval du capteur de température (67) à un endroit auquel, par rapport à la pression de l'air au niveau de l'entrée du four continu (1), il règne une pression d'air inférieure, et en particulier à un endroit sur la trajectoire d'écoulement du côté d'entrée du sas filtrant (5).
